# EUROPÄISCHE PATENTSCHRIFT

(11) **EP 1 503 976 B1**
(45) Veröffentlichungstag und Bekanntmachung des Hinweises auf die Patenterteilung: **31.03.2010**
(21) Anmeldenummer: 03747126.5
(22) Anmeldetag: 25.04.2003
(51) Int. Cl.: C07C 43/11, C11D 1/722

(54) **C sb 10 /sb -ALKANOLALKOXYLAT-GEMISCHE UND IHRE VERWENDUNG**
C sb 10 /sb -ALKANOLALKOXYLATE MIXTURES AND THE USE THEREOF
MELANGES DE C SB 10 /SB -ALCANOLALCOXYLATES ET LEUR UTILISATION

(30) Priorität: 26.04.2002 DE 10218753; 18.09.2002 DE 10243363
(43) Veröffentlichungstag der Anmeldung: 09.02.2005
(73) Patentinhaber: BASF SE, 67056 Ludwigshafen (DE)
(72) Erfinder: RULAND, Alfred, 69198 Schriesheim (DE); SCHOLTISSEK, Martin, 67157 Wachenheim (DE); TROPSCH, Jürgen, 67354 Römerberg (DE); BÖHN, Roland, 67136 Fussgönheim (DE); HACKMANN, Claus, 67281 Kirchheim (DE); WULFF, Christian, 68163 Mannheim (DE)
(86) Internationale Anmeldenummer: PCT/EP2003/004335
(87) Internationale Veröffentlichungsnummer: WO 2003/091192

(56) Entgegenhaltungen:
- WO-A-94/11331

## Beschreibung

Die vorliegende Erfindung betrifft die Verwendung von C₁₀-Alkanolalkoxylat-Gemischen, derartige C₁₀-Alkanolalkoxylat-Gemische und Verfahren zu ihrer Herstellung.

Alkoxylate von aliphatischen Alkoholen werden in großem Umfang als Tenside, Emulgatoren oder Schaumdämpfer eingesetzt. Die Benetzungs- und Emulgatoreigenschaften hängen dabei stark von der Art des Alkohols und der Art und Menge der Alkoxid-Addukte ab.

WO 94/11331 betrifft die Verwendung von Alkoxylaten von 2-Propylheptanol in Detergenzzusammensetzungen zur Entfettung harter Oberflächen. Die Alkoxylate weisen 2 bis 16 Alkylenoxid-Gruppen auf. Vorzugsweise liegt der überwiegende Teil der Alkylenoxid-Gruppen in Form von Ethylenoxid vor. Gemäß der Beispiele werden ausschließlich ethoxylierte Alkohole eingesetzt. Es ist ferner beschrieben, dass die Alkohole zunächst mit Ethylenoxid und sodann mit Propylenoxid umgesetzt werden können. Für derartige Alkoxylate sind jedoch keine Beispiele oder Eigenschaften angegeben. Es wird ausgeführt, dass die beschriebenen Alkoxylate eine gute Detergenzund Benetzungswirkung zeigen, verbunden mit einem geringen Schäumen. Zudem wird angegeben, dass die Alkoxylate einen erwünschten Verdickungseffekt in Formulierungen haben.

WO 94/11330 betrifft Alkoxylate von 2-Propylheptanol und deren Verwendung. In den Alkoxylaten liegt 2-Propylheptanol, zunächst mit 1 bis 6 mol Propylenoxid und sodann mit 1 bis 10 mol Ethylenoxid umgesetzt, vor. Gemäß den Beispielen wird ein zunächst mit 4 mol Propylenoxid und sodann mit 6 mol Ethylenoxid umgesetztes 2-Propylheptanol eingesetzt. Es wird angegeben, dass die Alkylenoxidaddukte ein verbessertes Verhältnis von Schaumverhalten zu Detergenzwirkung zeigen. Ferner ist angegeben, dass die Alkoxylate ein gutes Benetzungsverhalten zeigen. Sie werden in Detergenzzusammensetzungen zur Reinigung von Textilmaterialien eingesetzt.

US 2,508,036 betrifft die Verwendung von 2-n-Propylheptanolethoxilaten, die 5 bis 15 mol Ethylenoxid enthalten, als Netzmittel in wässrigen Lösungen. Es ist beschrieben, dass die Produkte als Tenside in Waschmitteln eingesetzt werden können. Verfahren zur Alkoxylierung von 2-Propylheptanol sind prinzipiell aus dem Stand der Technik bekannt. In der WO 01/04183 wird beispielsweise ein Verfahren zur Ethoxylierung von hydroxyfunktionellen Starterverbindungen beschrieben, das in Gegenwart einer Doppelmetallcyanid-Verbindung als Katalysator durchgeführt wird.

Aufgabe der vorliegenden Erfindung ist die Bereitstellung von Alkanolalkoxylaten, die als Emulgator, Schaumregulierer und als Netzmittel für harte Oberflächen geeignet sind. Die Alkoxylate sollen insbesondere ein gutes Emulgierverhalten und einen geringen Kontaktwinkel auf harten Oberflächen bei der Anwendung zeigen. Ferner sollen sie die Grenzflächenspannung in flüssigen Systemen vermindern. Die Alkoxylate sollen allgemein ein vorteilhaftes Eigenschaftsspektrum bei der Verwendung als Emulgator, Schaumregulierer oder als Netzmittel zeigen. Des weiteren sollte die Menge an Restalkohol reduziert werden, um Geruchsbeeinträchtigungen zu vermeiden.

Die Aufgabe wird erfindungsgemäß gelöst durch Alkoxylat-Gemische, enthaltend Alkoxylate der allgemeinen Formel (I)

C₅H₁₁CH(C₃H₇)CH₂O(A)ₙ(B)ₘH (I)

mit der Bedeutung
- A: Ethylenoxy
- B: C₃₋₁₀-Alkylenoxy, vorzugsweise Propylenoxy, Butylenoxy, Pentylenoxy oder Gemische davon,
wobei Gruppen A und B statistisch verteilt, alternierend oder in Form zweier oder mehrerer Blöcke in beliebiger Reihenfolge vorliegen können,
- n: Zahl von 0 bis 30,
- m: Zahl von 0 bis 20
- n + m: mindestens 1
wobei

| | |
|---|---|
| 70 bis 99 Gew.-% | Alkoxylate A1, in denen C₅H₁₁ die Bedeutung n-C₅H₁₁ hat, und |
| 1 bis 30 Gew.-% | Alkoxylate A2, in denen C₅H₁₁ die Bedeutung C₂H₅CH(CH₃)CH₂ und/oder CH₃CH(CH₃)CH₂CH₂ hat, |

im Gemisch vorliegen.

Es wurde erfindungsgemäß gefunden, dass die vorstehenden Alkoxylat-Gemische hervorragende Emulgatoreigenschaften zeigen und als nicht oder wenig schäumende Netzmittel für harte Oberflächen eingesetzt werden können. Die Alkoxylate zeigen geringe Kontaktwinkel bei der Benetzung harter Oberflächen und erlauben die Einstellung geringer Grenzflächenspannungen in flüssigen Systemen.

Damit sind die Alkoxylat-Gemische der allgemeinen Formel (I) besonders vorteilhaft einsetzbar, insbesondere als Emulgator, Schaumregulierer und als Netzmittel für harte Oberflächen in Tensidformulierungen zur Reinigung harter Oberflächen, in Feuchthaltemitteln, kosmetischen, pharmazeutischen und Pflanzenschutzformulierungen, Lacken, Beschichtungsmitteln, Klebstoffen, Lederentfettungsmitteln, Formulierungen für die Textilindustrie, Faserverarbeitung, Metallverarbeitung, Lebensmittelindustrie, Wasserbehandlung, Papierindustrie, Fermentation, Mineralverarbeitung und in Emulsionspolymerisationen. Auf die einzelnen Anwendungsgebiete wird nachfolgend noch näher eingegangen.

In der allgemeinen Formel (I) bedeutet n vorzugsweise eine Zahl im Bereich von 0 bis 30, insbesondere von 3 bis 12 m ist vorzugsweise eine Zahl im Bereich von 0 bis 8, insbesondere 1 bis 8, besonders bevorzugt 1 bis 5. B ist vorzugsweise Propylenoxy und/oder Butylenoxy.

In den erfindungsgemäßen Alkoxylaten können an den Alkoholrest anschließend zunächst Propylenoxy-Einheiten und daran anschließend Ethylenoxy-Einheiten vorliegen. Haben n und m einen Wert von mehr als 1, so liegen die entsprechenden Alkoxyreste vorzugsweise in Blockform vor. n und m bezeichnen dabei einen mittleren Wert, der sich als Durchschnitt für die Alkoxylate ergibt. Daher können n und m auch von ganzzahligen Werten abweichen. Bei der Alkoxylierung von Alkanolen wird im Allgemeinen eine Verteilung des Alkoxylierungsgrades erhalten, die in gewissem Umfang durch Einsatz unterschiedlicher Alkoxylierungskatalysatoren eingestellt werden kann. In den erfindungsgemäßen Alkoxylat-Gemischen können auch an den Alkoholrest anschließend zunächst Ethylenoxy-Einheiten und daran anschließend Propylenoxy-Einheiten vorliegen. Ferner können statistische Gemische von Ethylenoxid-Einheiten und Propylenoxid-Einheiten vorliegen. Auch 3- oder Mehrblockalkoxylierung und gemischte Alkoxylierung sind möglich. Es ist ferner auch möglich, dass nur Ethylenoxid-Einheiten A oder nur Einheiten B, insbesondere Propylenoxid-Einheiten, vorliegen. Durch die Auswahl geeigneter Mengen der Gruppen A und B kann das Eigenschaftsspektrum der erfindungsgemäßen Alkoxylat-Gemische je nach praktischen Erfordernissen angepasst werden. Besonders bevorzugt wird zunächst mit Propylenoxid, Butylenoxid, Pentenoxid oder Gemischen davon und anschließend mit Ethylenoxid umgesetzt. Ebenso ist es jedoch möglich, dass die Umsetzung mit Ethylenoxid alleine stattfindet.

Besonders bevorzugt bedeutet in der allgemeinen Formel (I) B Propylenoxy. n ist dann besonders bevorzugt eine Zahl von 1 bis 20 m ist besonders bevorzugt eine Zahl von 1 bis 8.

Die erfindungsgemäßen Alkoxylat-Gemische werden durch Alkoxylierung der zugrundeliegenden Alkohole C₅H₁₁CH(C₃H₇)CH₂OH erhalten. Die Ausgangsalkohole können aus den einzelnen Komponenten gemischt werden, so dass sich das erfindungsgemäße Verhältnis ergibt. Sie können durch Aldolkondensation von Valeraldehyd und nachfolgende Hydrierung hergestellt werden. Die Herstellung von Valeraldehyd und den entsprechenden Isomeren erfolgt durch Hydroformylierung von Buten, wie beispielsweise in US 4,287,370; Beilstein E IV 1, 32 68, Ullmanns Encyclopedia of Industrial Chemistry, 5. Auflage, Band A1, Seiten 323 und 328 f beschrieben. Die nachfolgende Aldolkondensation ist beispielsweise beschrieben in US 5,434,313 und Römpp, Chemie Lexikon, 9. Auflage, Stichwort "Aldol-Addition" Seite 91. Die Hydrierung des Aldolkondensationsproduktes folgt allgemeinen Hydrierbedingungen.

Des weiteren kann 2-Propylheptanol durch Kondensation von 1-Pentanol (als Mischung der entsprechenden Methylbutanole-1) in Gegenwart von KOH bei erhöhten Temperaturen hergestellt werden, siehe z.B. Marcel Guerbet, C.R Acad Sci Paris 128, 511, 1002 (1899). Des weiteren ist auf Römpp, Chemie Lexikon, 9. Auflage, Georg Thieme Verlag Stuttgart, und die dort genannten Ziate sowie Tetrahedron, Vol. 23, Seiten 1723 bis 1733, hinzuweisen.

In der allgemeinen Formel (I) kann der Rest C₅H₁₁ die Bedeutung n-C₅H₁₁, C₂H₅CH(CH₃)CH₂ oder CH₃CH(CH₃)CH₂CH₂ haben. Es handelt sich bei den Alkoxylaten um Gemische, wobei
70 bis 99 Gew.-%, vorzugsweise 85 bis 96 Gew.-% Alkoxylate A1 vorliegen, in denen C₅H₁₁ die Bedeutung n-C₅H₁₁ hat, und 1 bis 30 Gew.-%, vorzugsweise 4 bis 15 Gew.-% Alkoxylate A2, in denen C₅H₁₁ die Bedeutung C₂H₅CH(CH₃)CH₂ und/oder CH₃CH(CH₃)CH₂CH₂ hat.

Der Rest C₃H₇ hat vorzugsweise die Bedeutung n-C₃H₇.

Vorzugsweise wird die Alkoxylierung durch starke Basen katalysiert, die zweckmäßigerweise in Form eines Alkalialkoholats, Alkalihydroxids oder Erdalkalihydroxids, in der Regel in einer Menge von 0,1 bis 1 Gew.-% bezogen auf die Menge des Alkanols R²-OH, zugesetzt werden, (vergl. G. Gee et al., J. Chem. Soc. (1961), S. 1345; B. Wojtech, Makromol. Chem. 66, (1966), S. 180).

Auch eine saure Katalyse der Additionsreaktion ist möglich. Neben Bronstedsäuren eignen sich auch Lewissäuren wie zum Beispiel AlCl₃ oder BF₃ Dietherat, BF₃, BF₃ x H₃PO₄, SbCl₄ x 2 H₂O, Hydrotalcit (Vgl. P.H. Plesch, The Chemistry of Cationic Polymerization, Pergamon Press, New York (1963). Geeignet als Katalysator sind auch Doppelmetallcyanid (DMC) Verbindungen.

Als DMC-Verbindung können prinzipiell alle dem Fachmann bekannten geeigneten Verbindungen verwendet werden.

Als Katalysator geeignete DMC-Verbindungen sind beispielsweise in der WO 99/16775 und der DE-A-101 17 273 beschrieben. Insbesondere sind für die Alkoxylierung Doppelmetallcyanid-Verbindung der allgemeinen Formel I als Katalysator geeignet:

M¹ₐ[M²(CN)_{b}(A)_{c}]_{d}·fM¹_{g}Xₙ·h(H₂O)·eL·kP (I),

in der
- M¹ mindestens ein Metallion, ausgewählt aus der Gruppe bestehend aus Zₙ²⁺, Fₑ²⁺, Fₑ³⁺, Cₒ³⁺, Ni²⁺, Mₙ²⁺, Co²⁺, Sₙ²⁺, Pb²⁺, Mo⁴⁺, Mo⁶⁺, Al³⁺, V⁴⁺, V⁵⁺ Sr²⁺, W⁴⁺, W⁶⁺, Cr²⁺, Cr³⁺, Cd²⁺, Hg²⁺, Pd²⁺, Pt²⁺, V²⁺, Mg²⁺, Ca²⁺, Ba²⁺ Cu²⁺, La³⁺, Ce³⁺, Ce⁴⁺, Eu³⁺, Ti³⁺, Ti⁴⁺, Ag⁺, Rh²⁺, Rh³⁺, Ru²⁺, Ru³⁺ ist,
- M² mindestens ein Metallion, ausgewählt aus der Gruppe bestehend aus Fe²⁺, Fe³⁺, Co²⁺, Co³⁺, Mn²⁺, Mn³⁺, V⁴⁺, V⁵⁺, Cr²⁺, Cr³⁺, Rh³⁺, Ru²⁺, Ir³⁺ ist,
- A und X unabhängig voneinander ein Anion, ausgewählt aus der Gruppe, bestehend aus Halogenid, Hydroxid, Sulfat, Carbonat, Cyanid, Thiocyanat, Isocyanat, Cyanat, Carboxylat, Oxalat, Nitrat, Nitrosyl, Hydrogensulfat, Phosphat, Dihydrogenphosphat, Hydrogenphosphat oder Hydrogencarbonat sind,
- L ein mit Wasser mischbarer Ligand ist, ausgewählt aus der Gruppe, bestehend aus Alkoholen, Aldehyden, Ketonen, Ethern, Polyethern, Estern, Polyestern, Polycarbonat, Harnstoffen, Amiden, primären, sekundären und tertiären Aminen, Liganden mit Pyridin-Stickstoff, Nitrilen, Sulfiden, Phosphiden, Phosphiten, Phosphanen, Phosphonaten und Phosphaten,
- k eine gebrochene oder ganze Zahl größer oder gleich Null ist, und
- P ein organischer Zusatzstoff ist,
- a, b, c, d, g und n so ausgewählt sind, dass die Elektroneutralität der Verbindung (I) gewährleistet ist, wobei c = 0 sein kann,
- e die Anzahl der Ligandenmoleküle eine gebrochenen oder ganze Zahl größer 0 oder 0 ist,
- f und h unabhängig voneinander eine gebrochene oder ganze Zahl größer 0 oder 0 sind.

Als organische Zusatzstoffe P sind zu nennen: Polyether, Polyester, Polycarbonate, Polyalkylenglykolsorbitanester, Polyakylenglykolglycidylether, Polyacrylamid, Poly(acrylamid-co-acrylsäure), Polyacrylsäure, Poly(acrylamid-co-maleinsäure), Polyacrylnitril, Polyalkylacrylate, Polyalkylmethacrylate, Polyvinylmethylether, Polyvinylethylether, Polyvinylacetat, Polyvinylalkohol, Poly-N-vinylpyrrolidon, Poly(N-vinylpyrrolidon-co-acrylsäure), Polyvinylmethylketon, Poly(4-vinylphenol), Poly(acrylsäure-co-styrol), Oxazolinpolymere, Polyalkylenimine, Maleinsäure- und Maleinsäureanhydridcopolymere, Hydroxyethylcellulose, Polyacetate, ionische oberflächen- und grenzflächenaktive Verbindungen, Gallensäure oder deren Salze, Ester oder Amide, Carbonsäureester mehrwertiger Alkohole und Glycoside.

Diese Katalysatoren können kristallin oder amorph sein. Für den Fall, dass k gleich null ist, sind kristalline Doppelmetallcyanid-Verbindungen bevorzugt. Im Fall, dass k größer null ist, sind sowohl kristalline, teilkristalline, als auch substantiell amorphe Katalysatoren bevorzugt.

Von den modifizierten Katalysatoren gibt es verschiedene bevorzugte Ausführungsformen. Eine bevorzugte Ausführungsform sind Katalysatoren der Formel (I), bei denen k größer null ist. Der bevorzugte Katalysator enthält dann mindestens eine Doppelmetallcyanid-Verbindung, mindestens einen organischen Liganden und mindestens einen organischen Zusatzstoff P.

Bei einer anderen bevorzugten Ausführungsform ist k gleich null, optional ist e auch gleich null und X ist ausschließlich ein Carboxylat, bevorzugt Formiat, Acetat und Propionat. Derartige Katalysatoren sind in der WO 99/16775 beschrieben. Bei dieser Ausführungsform sind kristalline Doppelmetallcyanid-Katalysatoren bevorzugt. Ferner bevorzugt sind Doppelmetallcyanid-Katalysatoren, wie in der WO 00/74845 beschrieben, die kristallin und plättchenförmig sind.

Die Herstellung der modifizierten Katalysatoren erfolgt durch Vereinigung einer Metallsalz-Lösung mit einer Cyanometallat-Lösung, die optional sowohl einen organischen Liganden L als auch einen organischen Zusatzstoff P enthalten können. Anschließend werden der organische Ligand und optional der organische Zusatzstoff zugegeben. Bei einer bevorzugten Ausführungsform der Katalysatorherstellung wird zunächst eine inaktive Doppelmetallcyanid-Phase hergestellt und diese anschließend durch Umkristallisation in eine aktive Doppelmetallcyanidphase überführt, wie in der PCT/EP01/01893 beschrieben.

Bei einer anderen bevorzugten Ausführungsform der Katalysatoren sind f, e und k ungleich Null. Dabei handelt es sich um Doppelmetallcyanid-Katalysatoren, die einen mit Wasser mischbaren organischen Ligand (im allgemeinen in Mengen von 0,5 bis 30 Gew.%) und einen organischen Zusatzstoff (im allgemeinen in Mengen von 5 bis 80 Gew.%) enthalten wie in der WO 98/06312 beschrieben. Die Katalysatoren können entweder unter starkem Rühren (24000U/Min mit Turrax) oder unter Rühren hergestellt werden wie in der US 5,158,922 beschrieben.

Insbesondere als Katalysator geeignet sind für die Alkoxylierung Doppelmetallcyanid-Verbindungen, die Zink, Kobalt oder Eisen oder zwei davon enthalten. Besonders geeignet ist beispielsweise Berliner Blau.

Bevorzugt werden kristalline DMC-Verbindungen eingesetzt. In einer bevorzugten Ausführungsform wird eine kristalline DMC-Verbindung vom Zn-Co-Typ als Katalysator verwendet, der als weitere Metallsalzkomponente Zinkacetat enthält. Derartige Verbindungen kristallisieren in monokliner Struktur und weisen einen plättchenförmigen Habitus auf. Derartige Verbindungen werden beispielsweise in der WO 00/74845 oder der PCT/EP01/01893 beschrieben.

Als Katalysator geeignete DMC-Verbindungen können prinzipiell auf alle dem Fachmann bekannten Arten hergestellt werden. Beispielsweise können die DMC-Verbindungen durch direkte Fällung, "incipient wetness"-Methode, durch Herstellung einer Precursor-Phase und anschließende Umkristallisation hergestellt werden.

Die DMC-Verbindungen können als Pulver, Paste oder Suspension eingesetzt werden oder zu einem Formkörper verformt werden, in Formkörpern, Schäume oder ähnliches eingebracht werden oder auf Formkörper, Schäume oder ähnliches aufgebracht werden.

Die zur Alkoxylierung eingesetzte Katalysator-Konzentration, bezogen auf das Endmengengerüst ist typischerweise kleiner als 2000 ppm (d.h. mg Katalysator pro kg Produkt), bevorzugt kleiner als 1000 ppm, insbesondere kleiner als 500 ppm, besonders bevorzugt kleiner als 100 ppm, beispielsweise kleiner als 50 ppm oder 35 ppm, insbesondere bevorzugt kleiner als 25 ppm.

Die Additionsreaktion wird bei Temperaturen von 90 bis 240°C, vorzugsweise von 120 bis 180°C, im geschlossenen Gefäß ausgeführt. Das Alkylenoxid oder die Mischung verschiedener Alkylenoxide wird der Mischung aus erfindungsgemäßen Alkanolgemisch und Alkali unter dem bei der gewählten Reaktionstemperatur herrschenden Dampfdruck des Alkylenoxidgemisches zugeführt. Gewünschtenfalls kann das Alkylenoxid mit bis zu etwa 30 bis 60 % mit einem Inertgas verdünnt werden. Dadurch wird eine zusätzliche Sicherheit gegen explosionsartige Polyaddition des Alkylenoxids gegeben.

Wird ein Alkylenoxidgemisch eingesetzt, so werden Polyetherketten gebildet, in denen die verschiedenen Alkylenoxidbausteine praktisch statistisch verteilt sind. Variationen in der Verteilung der Bausteine längs der Polyetherkette ergeben sich aufgrund unterschiedlicher Reaktionsgeschwindigkeiten der Komponenten und können auch willkürlich durch kontinuierliche Zufuhr einer Alkylenoxidmischung programmgesteuerter Zusammensetzung erreicht werden. Werden die verschiedenen Alkylenoxide nacheinander zur Reaktion gebracht, so erhält man Polyetherketten mit blockartiger Verteilung der Alkylenoxid-Bausteine.

Die Länge der Polyetherketten schwankt innerhalb des Reaktionsprodukts statistisch um einen Mittelwert, der im Wesentlichen sich aus der Zusatzmenge ergebenden stöchiometrischen Wert.

Bevorzugte Alkoxylat-Gemische der allgemeinen Formel (I) können erfindungsgemäß erhalten werden durch Umsetzung von Alkoholen der allgemeinen Formel C₅H₁₁CH(C₃H₇)CH₂OH zuerst mit Propylenoxid und sodann mit Ethylenoxid unter Alkoxylierungsbedingungen oder nur mit Ethylenoxid. Geeignete Alkoxylierungsbedingungen sind vorstehend und in Nikolaus Schönfeldt, Grenzflächenaktive Äthylenoxid-Addukte, Wissenschaftliche Verlagsgesellschaft mbH Stuttgart 1984 beschrieben. In der Regel wird die Alkoxylierung in Gegenwart basischer Katalysatoren wie KOH in Substanz durchgeführt. Die Alkoxylierung kann jedoch auch unter Mitverwendung eines Lösungsmittels durchgeführt werden. Zur Herstellung dieser erfindungsgemäßen Alkoxylat-Gemische werden die Alkohole zunächst mit einer geeigneten Menge an Propylenoxid und sodann mit einer geeigneten Menge an Ethylenoxid umgesetzt oder nur mit Ethylenoxid. Dabei wird eine Polymerisation des Alkylenoxids in Gang gesetzt, bei der es zwangsläufig zu einer statistischen Verteilung von Homologen kommt, deren Mittelwert vorliegend mit n und m angegeben wird.

Durch die erfindungsgemäß bevorzugt zunächst durchgeführte Propoxylierung und erst nachfolgende Ethoxylierung kann der Gehalt an Restalkohol in den Alkoxylaten vermindert werden, da Propylenoxid gleichmäßiger an die Alkoholkomponente addiert wird. Im Unterschied dazu reagiert Ethylenoxid vorzugsweise mit Ethoxylaten, so dass bei einer anfänglichen Verwendung von Ethylenoxid zur Umsetzung mit den Alkanolen sowohl eine breite Homologenverteilung als auch ein hoher Gehalt an Restalkohol resultieren. Die Vermeidung von größeren Mengen an im Produkt vorliegendem Restalkohol ist insbesondere aus Geruchsgründen vorteilhaft. Die erfindungsgemäß eingesetzten Alkohol-Gemische haben in der Regel einen Eigengeruch, der durch die vollständige Alkoxylierung weitestgehend unterdrückt werden kann. Nach üblichen Verfahren erhaltene Alkoxylate weisen oftmals einen Eigengeruch auf, der für viele Anwendungen störend ist.

Überraschenderweise wurde gefunden, dass dieser Effekt bereits bei der Verwendung von geringen Mengen an Propylenoxid auftritt, also erfindungsgemäß weniger als 1,5 Äquivalente, bezogen auf den eingesetzten Alkohol, insbesondere weniger als 1,2 Äquivalente, besonders bevorzugt weniger als 1 Äquivalent.

Die erfindungsgemäßen Alkoxylat-Gemische erfordern zum Absenken des Restalkohol-Gehaltes nur einen direkt an den Alkohol gebundenen Propylenoxid(PO)-Block von sehr kurzer Länge. Dies ist insbesondere deshalb sehr vorteilhaft, weil die biologische Abbaubarkeit des Produktes bei Verlängerung des PO-Blocks sinkt. Derartige Alkoxylat-Gemische ermöglichen somit maximale Freiheitsgrade bei der Wahl der Länge des PO-Blockes, wobei die Länge nach unten durch den steigenden Restalkoholgehalt und nach oben durch die Verschlechterung der biologischen Abbaubarkeit begrenzt ist. Dies ist besonders dann vorteilhaft, wenn auf den PO-Block nur ein kurzer Ethylenoxid Block folgt.

Daher ist es im Rahmen der vorliegenden Erfindung weiter bevorzugt, dass m eine ganze oder gebrochene Zahl mit 0 < m ≤ 5, beispielsweise 0 < m ≤ 2, bevorzugt 0 < m ≤ 1,5, besonders bevorzugt 0 < m ≤ 1,2, insbesondere 0 < m < 1 ist.

Es ist erfindungsgemäß nicht notwendig, dass ein großer Restgehalt an Alkohol in den erfindungsgemäßen Alkoxylat-Gemischen vorliegt. Gemäß einer Ausführungsform der Erfindung weisen die Alkoxylat-Gemische einen verminderten Anteil an Alkoholen auf.

Die erfindungsgemäßen Alkoxylat-Gemische zeigen eine verbesserte Netzung auf harten Oberflächen.

Das vorteilhafte Netzungsverhalten der erfindungsgemäßen Gemische kann beispielsweise durch Messungen des Kontaktwinkels auf Glas, Polyethylenoxid oder Stahl ermittelt werden. Aus dem verbesserten Netzungsverhalten folgt eine bessere Performance bei insbesondere schnellen Reinigungsprozessen. Dies ist insofern überraschend, da durch die Kettenverlängerung des Ausgangsalkohols üblicherweise die dynamischen und netzenden Eigenschaften vermindert werden. Mit den erfindungsgemäßen Alkoxylat-Gemischen kann damit die Benetzungsgeschwindigkeit von wässrigen Formulierungen erhöht werden. Die erfindungsgemäßen Alkoxylat-Gemische können damit auch als Solubilisatoren eingesetzt werden, die insbesondere das Netzvermögen von Netzhilfsmitteln auch in verdünnten Systemen nicht negativ, sondern positiv beeinflussen. Sie können zur Erhöhung der Löslichkeit von Netzhilfsmitteln in wässrigen Formulierungen eingesetzt werden, die nicht-ionische Tenside enthalten. Sie dienen insbesondere zur Erhöhung der Benetzungsgeschwindigkeit in wässrigen Netzmitteln.

Ferner dienen die erfindungsgemäßen Alkoxylat-Gemische zur Verminderung der Grenzflächenspannung, beispielsweise in wässrigen Tensidformulierungen. Die verminderte Grenzflächenspannung kann beispielsweise durch die Pendant-Drop-Methode bestimmt werden. Hieraus ergibt sich auch eine bessere Wirkung der erfindungsgemäßen Alkoxylat-Gemische als Emulgator oder Co-Emulgator. Die erfindungsgemäßen Alkoxylat-Gemische können auch zur Verminderung der Grenzflächenspannung bei kurzen Zeiten von üblicherweise unter einer Sekunde bzw. zur Beschleunigung der Einstellung der Grenzflächenspannung in wässrigen Tensidformulierungen eingesetzt werden.

Die vorliegende Erfindung betrifft auch Reinigungs-, Netz-, Beschichtungs-, Klebe-, Lederentfettungs-, Feuchthalte- oder Textilbehandlungsmittel oder kosmetische, pharmazeutische oder Pflanzenschutzformulierungen, die mindestens ein wie vorstehend definiertes Alkoxylat-Gemische der allgemeinen Formel (I) enthalten. Die Mittel enthalten dabei vorzugsweise 0,1 bis 20 Gew.-% der Alkoxylat-Gemische. Nachstehend werden bevorzugte Einsatzgebiete der erfindungsgemäßen Alkoxylat-Gemische näher beschrieben.

Die erfindungsgemäßen Alkoxylat-Gemische werden vorzugsweise in den folgenden Bereichen eingesetzt:
- Tensidformulierungen zur Reinigung harter Oberflächen: Geeignete Tensidformulierungen, die mit den erfindungsgemäßen Alkoxylaten additiviert werden können, sind beispielsweise in Formulating Detergents and Personal Care Products von Louis Ho Tan Tai, AOCS Press, 2000 beschrieben.
   Sie enthalten beispielsweise als weitere Komponenten Seife, anionische Tenside wie LAS oder Paraffinsulfonate oder FAS oder FAES, Säure wie Phosphorsäure, Amidosulfonsäure, Zitronensäure, Milchsäure, Essigsäure, andere organische und anorganische Säuren, Lösungsmittel wie Ethylenglykol, Isopropanol, Komplexbildner wie EDTA, NTA, MGDA, Phosphonate, Polymere wie Polyacrylate, Copolymere Maleinsäure-Acrylsäure, Alkalispender wie Hydroxide, Silicate, Carbonate, Parfümöle, Oxidationsmittel wie Perborate, Persäuren oder Trichloroisocyanursäure, Na- oder K-Dichlorisocyanurate, Enzyme; siehe auch Milton J. Rosen, Manilal Dahanayake, Industrial Utilization of Surfactants, AOCS Press, 2000 und Nikolaus Schönfeldt, Grenzflächenaktive Ethylenoxidaddukte. Hier sind auch Formulierungen für die anderen genannten Anwendungen im Prinzip abgehandelt. Es kann sich um Haushaltsreiniger wie Allzweckreiniger, Geschirrspülmittel für manuelles wie automatisches Geschirrspülen, Metallentfettung, Industrielle Applikationen wie Reinigungsmittel für die Nahrungsmittelindustrie Flaschenwäsche, etc. handeln. Es kann sich auch um Druckwalzen- und Druckplattenreinigungsmittel in der Druckindustrie handeln.
   Geeignete weitere Inhaltsstoffe sind dem Fachmann bekannt.
- Feuchthaltemittel, insbesondere für die Druckindustrie.
- Kosmetische, pharmazeutische und Pflanzenschutzformulierungen. Geeignete Pflanzenschutzformulierungen sind beispielsweise in der EP-A-0 050 228 beschrieben. Es können für Pflanzenschutzmittel übliche weitere Inhaltsstoffe vorliegen.
- Lacke, Beschichtungsmittel, Farben, Pigmentpräparationen sowie Klebstoffe in der Lack- und Folienindustrie.
- Lederentfettungsmittel.
- Formulierungen für die Textilindustrie wie Egalisiermittel oder Formulierungen zur Garnreinigung.
- Faserverarbeitung und Hilfsmittel für die Papier- und Zellstoffindustrie.
- Metallverarbeitung wie Metallveredelung und Galvanobereich.
- Lebensmittelindustrie.
- Wasserbehandlung und Trinkwassergewinnung.
- Fermentation.
- Mineralverarbeitung und Staubkontrolle.
- Bauhilfsmittel.
- Emulsionspolymerisation und Herstellung von Dispersionen.
- Kühl- und Schmiermittel.

Solche Formulierungen enthalten üblicherweise Inhaltsstoffe wie Tenside, Gerüst-, Duft- und Farbstoffe, Komplexbildner, Polymere und andere Inhaltsstoffe. Typische Formulierungen sind beispielsweise in WO 01/32820 beschrieben. Weitere für unterschiedliche Anwendungen geeignete Inhaltsstoffe sind in EP-A-0 620 270, WO 95/27034, EP-A-0 681 865, EP-A-0 616 026, EP-A-0 616 028, DE-A-42 37 178 und US 5,340,495 und in Schönfeldt, s.o., beispielhaft beschrieben.

Allgemein können die erfindungsgemäßen Alkoxylat-Gemische in allen Bereichen eingesetzt werden, in denen die Wirkung von grenzflächenaktiven Stoffen notwendig ist.

Die erfindungsgemäßen Strukturen weisen eine gegenüber bekannten Strukturen, geringe Aquatoxizität und leichte biologische Abbaubarkeit auf, so dass sie für eine Vielzahl von Anwendungsgebieten vorteilhaft geeignet sind.

Im folgenden soll die vorliegende Erfindung anhand von Beispielen näher erläutert werden.

### BEISPIELE

### Herstellbeispiel 1: DMC-Katalysator

In einem Rührkessel mit einem Volumen von 301, ausgestattet mit einem Propellerrührer, Tauchrohr für die Dosierung, pH-Sonde und Streulicht-Sonde, wurden 16000 g wässrige Hexacyanocobaltsäure (Cobalt-Gehalt: 9 g/l) vorgelegt und unter Rühren auf 50°C erwärmt. Anschließend wurden unter Rühren mit einer Rührleistung von 0,4 W/l 9224 g wässrige Zinkacetat-Dihydrat-Lösung (Zink-Gehalt: 2,6 Gew.-%), welche auf ebenfalls 50°C temperiert war, innerhalb von 15 Minuten zugefahren.

Zu dieser Fällsuspension wurden 351 g Pluronic® PE 6200 (BASF AG) zugesetzt und die Mischung weitere 10 Minuten gerührt.

Anschließend wurden weitere 3690 g wässrige Zinkacetat-Dihydrat-Lösung (Zink-Gehalt: 2,6 Gew.-%) unter Rühren mit einer Rührenergie von 1 W/l innerhalb 5 Minuten zudosiert.

Die Suspension wurde zwei Stunden nachgerührt. Der pH-Wert fiel in dieser Zeit von 4,02 auf 3,27 und blieb dann konstant. Die so erhaltene Fällsuspension wurde anschließend abfiltriert und auf dem Filter mit dem 6-fachen Kuchenvolumen an Wasser gewaschen.

Der feuchte Filterkuchen wurde getrocknet und mittels Spalt-Rotor-Mühle in Tridekanol® N dispergiert. Die dabei erhaltene Suspension hatte einen Multimetallcyanidgehalt von 5 Gew.-%.

### Beispiel 1 (2-Propylheptanol + 5 EO, 25 ppm DMC)

474 g (3,0 Mol) 2-Propyl-Heptanol-1 (Isomerengemisch aus 87% 2-Propylheptanol-1, 11 % 2-Propyl-4-methylhexanol-1, < 1% 2-Propyl-5-methylhexanol-1) und 0,567 g einer 5%-igen Suspension von Doppelmetallcyanid in 2-Propylheptanol-Isomerengemisch (25 ppm bezogen auf das Produkt) als Katalysator wurden bei einer Temperatur von 80 °C und ca. 1 mbar entwässert, anschließend in einem 21 Druckautoklaven vorgelegt, dreimal mit Stickstoff gespült und danach auf 120 °C erhitzt. Nach Erreichen der Temperatur wurden in 1,05 Stunden 660 g (15 Mol) Ethylenoxid kontinuierlich bei einem Druck von 0,1 bis 3,7 bar zudosiert (Druckrampe 6 bar/ 90 min). Nach vollständiger Oxidzugabe wurde bis zur Druckkonstanz nachreagieren lassen (20 Minuten), danach auf 80 °C abgekühlt, dreimal mit Stickstoff gespült und entleert. Das so erhaltene Produkt wurde bei 80 °C am Rotationsverdampfer unter Vakuum (< 30 mbar) entgast (Reaktionsprodukt nicht filtriert).

### Beispiel 2 (2-Propylheptanol + 3 EO, 25 ppm DMC)

Die Umsetzung wurde analog Beispiel 1 durchgeführt mit 474 g (3,0 Mol) 2-Propylheptanol-Isomerengemisch, 0,44 g Doppelmetallcyanid-Suspension und 397 g (9,0 Mol) Ethylenoxid.

### Beispiel 3 (2-Propylheptanol + 8 EO, 25 ppm DMC)

Die Umsetzung wurde analog Beispiel 1 durchgeführt mit 474 g (3,0 Mol) 2-Propylheptanol-Isomerengemisch, 0,77 g Doppelmetallcyanid-Suspension und 1060 g (24,0 Mol) Ethylenoxid.

### Herstellbeispiel 2: Synthese von Ethoxylaten des 2-Propylheptanols mittels Koch-katalyse

2-Propylheptanol und KOH (fein gepulvert) wurden vermischt und bei 80°C und 40 mbar über 1 Stunde entwässert. Das Reaktionsprodukt wurde in einen Autoklaven gegeben, der Autoklav 2 mal mit Stickstoff inertisiert und dann auf 120°C erwärmt. Innerhalb von 15 Minuten wurde Ethylenoxid bis zu einem Maximaldruck von 1 bar zudosiert. Man hielt 5 min bei diesem Druck, steigerte den Druck dann durch Zugabe von Ethylenoxid innerhalb 60 min auf 3 bar, hält 5 Stunden bei diesem Druck und steigerte schließlich den Druck bis auf 6 bar. Bei der letzten Dosierung wurde nur soviel Ethylenoxid zugegeben, bis die gewünschte Menge Ethylenoxid erreicht war. Der Druck wurde dann durch Zudosierung von Stickstoff bei 6 bar gehalten. Nach weiteren 10 Stunden Reaktionszeit wurde auf 80 °C abkühlen gelassen, und der Reaktionsaustrag ausgefüllt. Am Rotationsverdampfer wurden flüchtige Anteile bei 30 mbar und 80°C entfernt.

### Beispiel 4 (2-Propylheptanol + 3 EO, KOH katalysiert)

Die Synthese erfolgte analog Herstellbeispiel 2. Es wurden 474 g 2-Propylheptanol (3,0 mol), 397 g Ethylenoxid (9,0 mol) und 1,8 g KOH verwendet.
a) Als Startalkohol wurde reines 2-PH, hergestellt durch Destillation des techn. Gemisches, mit einer Reinheit von grösser 99 % eingesetzt. Das Produkt wies folgende Eigenschaften auf:

| | |
|---|---|
| Netzung auf textilen Oberflächen (EN 1772): | 13 sec (23 °C, 1 g/l in 2 g Soda/l) |
| Schäumvermögen (EN 12728): | ca. 20 ml (40 °C; 2 g/l; 1,8 mmol Ca2+-Ionen, nach 30 sec) |
| Oberflächenspannung (DIN 53914): | ca. 26,8 mN/m (1 g/l; 23 °C) |
| Ethoxilierungsgrad lt. OHZ: | 3,1 mol EO |

b) Als Startalkohol wurde 2-Propylheptanol, techn. Qualität mit ca. 90 % 2-Ph und ca. 10 % 4-Methyl-2-Propylhexanol eingesetzt. Das Produkt wies folgende Eigenschaften auf:

| | |
|---|---|
| Netzung auf textilen Oberflächen (EN 1772): | 12 sec (23 °C, 1 g/l in 2 g Soda/l) |
| Schäumvermögen (EN 12728): | ca. 20 ml (40 °C; 2 g/l; 1,8 mmol Ca2+-Ionen, nach 30 sec) |
| Oberflächenspannung (DIN 53914): | ca. 27,2 mN/m (1 g/l; 23 °C) |
| Ethoxilierungsgrad lt. OHZ: | 2,8 mol EO |

### Beispiel 5 (2-Propylheptanol + 5 EO, KOH katalysiert)

Die Synthese erfolgte analog Herstellbeispiel 2. Es wurden 474 g 2-Propylheptanol (3,0 mol), 661 g Ethylenoxid (15,0 mol) und 2,3 g KOH verwendet.
a) Als Startalkohol wurde reines 2-PH, hergestellt durch Destillation des techn. Gemisches, mit einer Reinheit von grösser 99 % eingesetzt. Das Produkt wies folgende Eigenschaften auf:

| | |
|---|---|
| Netzung auf textilen Oberflächen (EN 1772): | 10 sec (23 °C, 1 g/l in 2 g Soda/l) |
| Schäumvermögen (EN 12728): | 25 ml (40°C; 2 g/l; 1,8 mmol Ca2+-Ionen, nach 30 sec) |
| Oberflächenspannung (DIN 53914): | ca. 27,1 mN/m (1 g/l; 23 °C) |
| Ethoxilierungsgrad lt. OHZ: | 5,2 mol EO |

b) Als Startalkohol wurde 2-Propylheptanol, techn. Qualität mit ca. 90 % 2-Ph und ca. 10 % 4-Methyl-2-Propylhexanol eingesetzt. Das Produkt wies folgende Eigenschaften auf:

| | |
|---|---|
| Netzung auf textilen Oberflächen (EN 1772): | 9 sec (23 °C, 1 g/l in 2 g Soda/l) |
| Schäumvermögen (EN 12728): | 30 ml (40 °C; 2 g/l; 1,8 mmol Ca2+-Ionen, nach 30 sec) |
| Oberflächenspannung (DIN 53914): | ca. 26,3 mN/m (1 g/l; 23 °C) |
| Ethoxilierungsgrad lt. OHZ: | 4,6 mol EO |

### Beispiel 6 (2-Propylheptanol + 7 EO, KOH katalysiert)

Die Synthese erfolgte analog Herstellbeispiel 2. Es wurden 474 g 2-Propylheptanol (3,0 mol), 925 g Ethylenoxid (21,0 mol) und 2,8 g KOH verwendet.
a) Als Startalkohol wurde reines 2-PH, hergestellt durch Destillation des techn. Gemisches, mit einer Reinheit von grösser 99 % eingesetzt. Das Produkt wies folgende Eigenschaften auf:

| | |
|---|---|
| Netzung auf textilen Oberflächen (EN 1772): | 14 sec (23 °C, 1 g/l in 2 g Soda/l) |
| Schäumvermögen (EN 12728): | 330 ml (40 °C; 2 g/l; 1,8 mmol Ca2+-Ionen, nach 30 sec) |
| Oberflächenspannung (DIN 53914): | ca. 27,8 mN/m (1 g/l; 23 °C) |
| Ethoxilierungsgrad lt. OHZ: | 7,4 mol EO |

b) Als Startalkohol wurde 2-Propylheptanol, techn. Qualität mit ca. 90 % 2-Ph und ca. 10 % 4-Methyl-2-Propylhexanol eingesetzt. Das Produkt wies folgende Eigenschaften auf:

| | |
|---|---|
| Netzung auf textilen Oberflächen (EN 1772): | 13 sec (23 °C, 1 g/l in 2 g Soda/l) |
| Schäumvermögen (EN 12728): | 350 ml (40 °C; 2 g/l; 1,8 mmol Ca2+-Ionen, nach 30 sec) |
| Oberflächenspannung (DIN 53914): | ca. 27,1 mN/m (1 g/l; 23 °C) |
| Ethoxilierungsgrad lt. OHZ: | 7,1 mol EO |

### Beispiel 7 (2-Propylheptanol + 10 EO, KOH katalysiert)

Die Synthese erfolgte analog Herstellbeispiel 2. Es wurden 474 g 2-Propylheptanol (3,0 mol), 1.322 g Ethylenoxid (30,0 mol) und 3,6 g KOH verwendet.
a) Als Startalkohol wurde reines 2-PH, hergestellt durch Destillation des techn. Gemisches, mit einer Reinheit von grösser 99 % eingesetzt. Das Produkt wies folgende Eigenschaften auf:

| | |
|---|---|
| Netzung auf textilen Oberflächen (EN 1772): | 47 sec (23 °C, 1 g/l in 2 g Soda/l) |
| Schäumvermögen (EN 12728): | 380 ml (40 °C; 2 g/l; 1,8 mmol Ca2+-Ionen, nach 3 0 sec) |
| Oberflächenspannung (DIN 53914): | 30,5 mN/m (1 g/l; 23 °C) |
| Ethoxilierungsgrad lt. OHZ: | 10,4 mol EO |

b) Als Startalkohol wurde 2-Propylheptanol, techn. Qualität mit ca. 90 % 2-Ph und ca. 10 % 4-Methyl-2-Propylhexanol eingesetzt. Das Produkt wies folgende Eigenschaften auf:

| | |
|---|---|
| Netzung auf textilen Oberflächen (EN 1772): | 40 sec (23 °C, 1 g/l in 2 g Soda/l) |
| Schäumvermögen (EN 12728): | 370 ml (40 °C; 2 g/l; 1,8 mmol Ca2+-Ionen, nach 3 0 sec) |
| Oberflächenspannung (DIN 53914): | ca. 30,7 mN/m (1 g/l; 23 °C) |
| Ethoxilierungsgrad lt. OHZ: | 10,2 mol EO |

Die physikochemischen Eigenschaften und Tests auf Netzung, Schaum etc. zeigen also vergleichbare Performance, unabhängig davon ob mit technischer oder isomerenreiner Qualität hergestellt.

### Beispiel 8: Alkoxylierung mit EO mit DMC-Katalysator

### 8.1 2-Propylheptanol + 3 EO

316 g (2,0 Mol) 2-Propyl-Heptanol-1 (Isomerengemisch aus 87% 2-Propylheptanol-1, 11 % 2-Propyl-4-methylhexanol-1, <1% 2-Propyl-5-methylhexanol-1) und 35 ppm Doppelmetallcyanid-Katalysator (bezogen auf das Produkt) wurden bei einer Temperatur von 100 °C und ca. 20 mbar für zwei Stunden in einem Druckautoklaven entwässert. Anschließend wurde dreimal mit Stickstoff gespült und danach auf 140 °C erhitzt. Nach Erreichen der Temperatur wurden unter Rühren insgesamt 264 g (6,0 Mol) Ethylenoxid zudosiert. Nach beendeter Ethylenoxid-Dosierung rührte man noch 1 h bei 140 °C, spülte dreimal mit Stickstoff, evakuierte dann zum Entgasen auf 20 mbar, kühlte danach auf 80 °C ab, und entleerte den Reaktor. Das Reaktionsprodukt wurde nicht filtriert.

| | |
|---|---|
| Restalkoholgehalt (2-Propyl-heptanol-1): | 9,6% |

### 8.2 2-Propylheptanol + 4 EO

Es wurde vorgegangen wie in Beispiel 8.1. Allerdings gab man 8,0 Mol Ethylenoxid statt 6,0 Mol zu.

| | |
|---|---|
| Restalkoholgehalt (2-Propyl-heptanol-1): | 5,8% |

### 8.3 2-Propylheptanol + 5 EO

Es wurde vorgegangen wie in Beispiel 8.1. Allerdings gab man 10,0 Mol Ethylenoxid statt 6,0 Mol zu.

| | |
|---|---|
| Restalkoholgehalt (2-Propyl-heptanol-1): | 2,7% |

### 8.4 2-Propylheptanol + 6 EO

Es wurde vorgegangen wie in Beispiel 8.1. Allerdings gab man 12,0 Mol Ethylenoxid statt 6,0 Mol zu.

| | |
|---|---|
| Restalkoholgehalt (2-Propyl-heptanol-1): | 1,4% |

### 8.5 2-Propylheptanol + 7 EO

Es wurde vorgegangen wie in Beispiel 8.1. Allerdings gab man 14,0 Mol Ethylenoxid statt 6,0 Mol zu.

| | |
|---|---|
| Restalkoholgehalt (2-Propyl-heptanol-1): | 0,8% |

### 8.6 2-Propylheptanol + 8 EO

Es wurde vorgegangen wie in Beispiel 8.1. Allerdings gab man 16,0 Mol Ethylenoxid statt 6,0 Mol zu.

| | |
|---|---|
| Restalkoholgehalt (2-Propyl-heptanol-1): | 0,3% |

### 8.7 2-Propylheptanol + 10 EO

Es wurde vorgegangen wie in Beispiel 8.1. Allerdings gab man 20,0 Mol Ethylenoxid statt 6,0 Mol zu.

| | |
|---|---|
| Restalkoholgehalt (2-Propyl-heptanol-1): | 0,2% |

### 8.8 2-Propylheptanol + 14 EO

Es wurde vorgegangen wie in Beispiel 8.1. Allerdings gab man 28,0 Mol Ethylenoxid statt 6,0 Mol zu.

| | |
|---|---|
| Restalkoholgehalt (2-Propyl-heptanol-1): | 0,1% |

### Beispiel 9: Alkoxylierung mit PO mit DMC-Katalysator

### 9.1 2-Propylheptanol + 0,8 PO

316 g (2,0 Mol) 2-Propyl-Heptanol-1 (Isomerengemisch aus 87% 2-Propylheptanol-1, 11% 2-Propyl-4-methylhexanol-1, <1% 2-Propyl-5-methylhexanol-1) und 35 ppm Doppelmetallcyanid-Katalysator (bezogen auf das Produkt) wurden bei einer Temperatur von 100 °C und ca. 20 mbar für zwei Stunden in einem Druckautoklaven entwässert. Anschließend wurde dreimal mit Stickstoff gespült und danach auf 140 °C erhitzt. Nach Erreichen der Temperatur wurden unter Rühren insgesamt 93 g (1,6 Mol) Propylenoxid bei 140 °C zudosiert. Nach Ende der PO-Dosierung rührte man noch 15 Minuten bei 140 °C, spülte dreimal mit Stickstoff, evakuierte dann zum Entgasen auf 20 mbar, kühlte danach auf 80 °C ab, und entleerte den Reaktor.

| | |
|---|---|
| Restalkoholgehalt (2-Propyl-heptanol-1): | 28,6% |

### 9.2 2-Propylheptanol + 1,0 PO

Es wurde vorgegangen wie in Beispiel 9.1. Allerdings gab man 2,0 Mol Propylenoxid statt 1,6 Mol zu und arbeitete bei einer Reaktionstemperatur von 140°C.

| | |
|---|---|
| Restalkoholgehalt (2-Propyl-heptanol-1): | 24,2% |

### 9.3 2-Propylheptanol + 1,20 PO

Es wurde vorgegangen wie in Beispiel 9.1. Allerdings gab man 2,4 Mol Propylenoxid statt 1,6 Mol zu und arbeitete bei einer Reaktionstemperatur von 160°C.

| | |
|---|---|
| Restalkoholgehalt (2-Propyl-heptanol-1): | 20,0% |

### 9.4 2-Propylheptanol + 1,20 PO

Es wurde vorgegangen wie in Beispiel 9.1. Allerdings gab man 2,4 Mol Propylenoxid statt 1,6 Mol zu und arbeitete bei einer Reaktionstemperatur von 140°C.

| | |
|---|---|
| Restalkoholgehalt (2-Propyl-heptanol-1): | 19,8% |

### 9.5 2-Propylheptanol + 1,23 PO

Es wurde vorgegangen wie in Beispiel 9.1. Allerdings gab man 2,46 Mol Propylenoxid statt 1,6 Mol zu und arbeitete bei einer Reaktionstemperatur von 120°C.

| | |
|---|---|
| Restalkoholgehalt (2-Propyl-heptanol-1): | 20,8% |

### 9.6 2-Propylheptanol + 1,28 PO

Es wurde vorgegangen wie in Beispiel 9.1. Allerdings gab man 2,56 Mol Propylenoxid statt 1,6 Mol zu und arbeitete bei einer Reaktionstemperatur von 140°C.

| | |
|---|---|
| Restalkoholgehalt (2-Propyl-heptanol-1): | 17,7% |

### 9.7 2-Propylheptanol + 1,30 PO

Es wurde vorgegangen wie in Beispiel 9.1. Allerdings gab man 2,6 Mol Propylenoxid statt 1,6 Mol zu und arbeitete bei einer Reaktionstemperatur von 140°C.

| | |
|---|---|
| Restalkoholgehalt (2-Propyl-heptanol-1): | 17,6% |

### 9.8 2-Propylheptanol + 1,40 PO

Es wurde vorgegangen wie in Beispiel 9.1. Allerdings gab man 2,8 Mol Propylenoxid statt 1,6 Mol zu und arbeitete bei einer Reaktionstemperatur von 140 °C.

| | |
|---|---|
| Restalkoholgehalt (2-Propyl-heptanol-1): | 15,8% |

### 9.9 2-Propylheptanol + 1,44 PO

Es wurde vorgegangen wie in Beispiel 9.1. Allerdings gab man 2,88 Mol Propylenoxid statt 1,6 Mol zu und arbeitete bei einer Reaktionstemperatur von 140°C.

| | |
|---|---|
| Restalkoholgehalt (2-Propyl-heptanol-1): | 14,8% |

### 9.10 2-Propylheptanol+ 1,51 PO

Es wurde vorgegangen wie in Beispiel 9.1. Allerdings gab man 3,02 Mol Propylenoxid statt 1,6 Mol zu und arbeitete bei einer Reaktionstemperatur von 120 °C.

| | |
|---|---|
| Restalkoholgehalt (2-Propyl-heptanol-1): | 15,0% |

### 9.11 2-Propylheptanol + 1,63 PO

Es wurde vorgegangen wie in Beispiel 9.1. Allerdings gab man 3,26 Mol Propylenoxid statt 1,6 Mol zu und arbeitete bei einer Reaktionstemperatur von 160°C.

| | |
|---|---|
| Restalkoholgehalt (2-Propyl-heptanol-1): | 10,1 % |

### 9.12 2-Propylheptanol + 1,71 PO

Es wurde vorgegangen wie in Beispiel 9.1. Allerdings gab man 3,42 Mol Propylenoxid statt 1,6 Mol zu und arbeitete bei einer Reaktionstemperatur von 120°C.

| | |
|---|---|
| Restalkoholgehalt (2-Propyl-heptanol-1): | 10,7% |

### Beispiel 10: Alkoxylierung mit PO und EO mit DMC-Katalysator

### 10.1 2-Propylheptanol + 0,8 PO + 3 EO

316 g (2,0 Mol) 2-Propyl-Heptanol-1 (Isomerengemisch aus 87% 2-Propylheptanol-1, 11% 2-Propyl-4-methylhexanol-1, <1% 2-Propyl-5-methylhexanol-1) und 35 ppm Doppelmetallcyanid-Katalysator (bezogen auf das Produkt) wurden bei einer Temperatur von 100 °C und ca. 20 mbar für zwei Stunden in einem Druckautoklaven entwässert. Anschließend wurde dreimal mit Stickstoff gespült und danach auf 140 °C erhitzt. Nach Erreichen der Temperatur wurden unter Rühren insgesamt 93 g (1,6 Mol) Propylenoxid bei 140 °C zudosiert. Nach Ende der PO-Dosierung rührte man noch 15 Minuten bei 140 °C und begann dann mit der Dosierung von insgesamt 264 g (6,0 Mol) Ethylenoxid. Nach beendeter Ethylenoxid-Dosierung rührte man noch 1 h bei 140 °C, spülte dreimal mit Stickstoff, evakuierte dann zum Entgasen auf 20 mbar, kühlte danach auf 80 °C ab, und entleerte den Reaktor. Das Reaktionsprodukt wurde nicht filtriert.

| | |
|---|---|
| Restalkoholgehalt (2-Propyl-heptanol-1): | 1,9% |

### 10.2 2-Propylheptanol + 1,0 PO + 3 EO

Es wurde vorgegangen wie in Beispiel 10.1. Allerdings gab man 2,0 Mol Propylenoxid und 6,0 Mol Ethylenoxid zu.

| | |
|---|---|
| Restalkoholgehalt (2-Propyl-heptanol-1): | 1,3% |

### 10.3 2-Propylheptanol + 1,2 PO + 3 EO

Es wurde vorgegangen wie in Beispiel 10.1. Allerdings gab man 2,4 Mol Propylenoxid und 6,0 Mol Ethylenoxid zu.

| | |
|---|---|
| Restalkoholgehalt (2-Propyl-heptanol-1): | 0,9% |

### 10.4 2-Propylheptanol + 0,8 PO + 4 EO

Es wurde vorgegangen wie in Beispiel 10.1. Allerdings gab man 1,6 Mol Propylenoxid und 8,0 Mol Ethylenoxid zu.

| | |
|---|---|
| Restalkoholgehalt (2-Propyl-heptanol-1): | 1,0% |

### 10.5 2-Propylheptanol + 1,0 PO + 4 EO

Es wurde vorgegangen wie in Beispiel 10.1. Allerdings gab man 2,0 Mol Propylenoxid und 8,0 Mol Ethylenoxid zu.

| | |
|---|---|
| Restalkoholgehalt (2-Propyl-heptanol-1): | 0,7% |

### 10.6 2-Propylheptanol + 1,3 PO + 4 EO

Es wurde vorgegangen wie in Beispiel 10.1. Allerdings gab man 2,6 Mol Propylenoxid und 8,0 Mol Ethylenoxid zu.

| | |
|---|---|
| Restalkoholgehalt (2-Propyl-heptanol-1): | 0,5% |

### 10.7 2-Propylheptanol + 0,6 PO + 5 EO

Es wurde vorgegangen wie in Beispiel 10.1. Allerdings gab man 1,2 Mol Propylenoxid und 10,0 Mol Ethylenoxid zu.

| | |
|---|---|
| Restalkoholgehalt (2-Propyl-heptanol-1): | 0,8% |

### 10.8 2-Propylheptanol + 1,2 PO + 5 EO

Es wurde vorgegangen wie in Beispiel 10.1. Allerdings gab man 2,4 Mol Propylenoxid und 10,0 Mol Ethylenoxid zu.

| | |
|---|---|
| Restalkoholgehalt (2-Propyl-heptanol-1): | 0,14% |

### 10.9 2-Propylheptanol + 1,0 PO + 6 EO

Es wurde vorgegangen wie in Beispiel 10.1. Allerdings gab man 2,0 Mol Propylenoxid und 12,0 Mol Ethylenoxid zu.

| | |
|---|---|
| Restalkoholgehalt (2-Propyl-heptanol-1): | 0,12% |

### 10.10 2-Propylheptanol + 1,2 PO + 6 EO

Es wurde vorgegangen wie in Beispiel 10.1. Allerdings gab man 2,4 Mol Propylenoxid und 12,0 Mol Ethylenoxid zu.

| | |
|---|---|
| Restalkoholgehalt (2-Propyl-heptanol-1): | 0,09% |

### 10.11 2-Propylheptanol + 1,3 PO + 6 EO

Es wurde vorgegangen wie in Beispiel 10.1. Allerdings gab man 2,6 Mol Propylenoxid und 12,0 Mol Ethylenoxid zu.

| | |
|---|---|
| Restalkoholgehalt (2-Propyl-heptanol-1): | 0,06% |

### 10.12 2-Propylheptanol + 1,2 PO + 7 EO

Es wurde vorgegangen wie in Beispiel 10.1. Allerdings gab man 2,4 Mol Propylenoxid und 14,0 Mol Ethylenoxid zu.

| | |
|---|---|
| Restalkoholgehalt (2-Propyl-heptanol-1): | 0,03% |

### 10.13 2-Propylheptanol + 1,3 PO + 8 EO

Es wurde vorgegangen wie in Beispiel 10.1. Allerdings gab man 2,6 Mol Propylenoxid und 16,0 Mol Ethylenoxid zu.

| | |
|---|---|
| Restalkoholgehalt (2-Propyl-heptanol-1): | 0,02% |

### 10.14 2-Propylheptanol + 1,0 PO + 10 EO

Es wurde vorgegangen wie in Beispiel 10.1. Allerdings gab man 2,0 Mol Propylenoxid und 20,0 Mol Ethylenoxid zu.

| | |
|---|---|
| Restalkoholgehalt (2-Propyl-heptanol-1): | 0,01% |

### 10.15 2-Propylheptanol + 1,0 PO + 14 EO

Es wurde vorgegangen wie in Beispiel 10.1. Allerdings gab man 2,0 Mol Propylenoxid und 28,0 Mol Ethylenoxid zu.

| | |
|---|---|
| Restalkoholgehalt (2-Propyl-heptanol-1): | 0,01% |

Ein Vergleich der jeweils aus Beispiel 8 und 9 rechnerisch ermittelten Werte für den Restalkoholgehalt eines Produkts enthaltend PO und EO mit den experimentell für Beispiel 10 ermittelten Werten zeigt deutlich, dass die anfängliche Propoxylierung und anschließende Ethoxylierung im Vergleich zum theoretisch erwarteten Wert zu einem deutlich verminderten Restalkoholgehalt führt.

## Patentansprüche

1. Alkoxylat-Gemische, enthaltend Alkoxylate der allgemeinen Formel (I)
C₅H₁₁CH(C₃H₇)CH₂O(A)ₙ(B)ₘH (I)
mit der Bedeutung
A Ethylenoxy
B C₃₋₁₀-Alkylenoxy oder Gemische davon,
wobei Gruppen A und B statistisch verteilt, alternierend oder in Form zweier oder mehrerer Blöcke in beliebiger Reihenfolge vorliegen können,
n Zahl von 0 bis 30,
m Zahl von 0 bis 20
n + m mindestens 1
wobei
| | |
|---|---|
| 70 bis 99 Gew.-% | Alkoxylate A1, in denen C₅H₁₁ die Bedeutung n-C₅H₁₁ hat, und |
| 1 bis 30 Gew.% | Alkoxylate A2, in denen C₅H₁₁ die Bedeutung C₂H₅CH(CH₃)CH₂ und/oder CH₃CH(CH₃)CH₂CH₂ hat, |
im Gemisch vorliegen.

2. Alkoxylat-Gemische nach Anspruch 1, **dadurch gekennzeichnet, dass** C₃H₇ die Bedeutung n-C₃H₇ hat.

3. Alkoxylat-Gemische nach Anspruch 1 oder 2, **dadurch gekennzeichnet, dass** in der allgemeinen Formel (I) B Propylenoxy bedeutet, n eine Zahl von 1 bis 20 ist und m eine Zahl von 1 bis 8 ist.

4. Alkoxylat-Gemische nach einem der Ansprüche 1 bis 3, **dadurch gekennzeichnet, dass** 85 bis 96 Gew.-% Alkoxylate A1 und 4 bis 15 Gew.-% Alkoxylate A2 vorliegen.

5. Verfahren zur Herstellung von Alkoxylat-Gemischen nach einem der Ansprüche 1 bis 4 durch Umsetzung des Alkoholgemisches mit C₂₋₅-Alkylenoxiden unter Alkoxylierungsbedingungen, wobei die Alkoxylierung in Gegenwart einer Doppelmetallcyanid-Verbindung als Katalysator erfolgen kann.

6. Verwendung von Alkoxylat-Gemischen gemäß einem der Ansprüche 1 bis 4 als Emulgator, Schaumregulierer und als Netzmittel für harte Oberflächen.

7. Verwendung nach Anspruch 6 in Waschmitteln, Tensidformulierungen zur Reinigung harter Oberflächen, Feuchthaltemitteln, kosmetischen, pharmazeutischen und Pflanzenschutzformulierungen, Lacken, Beschichtungsmitteln, Klebstoffen, Lederentfettungsmitteln, Formulierungen für die Textilindustrie, Faserverarbeitung, Metallverarbeitung, Lebensmittelindustrie, Wasserbehandlung, Papierindustrie, Fermentation oder Mineralverarbeitung und in Emulsionspolymerisationen.

8. Wasch-, Reinigungs-, Netz-, Beschichtungs-, Klebe-, Lederentfettungs-, Feuchthalte- oder Textilbehandlungsmittel oder kosmetische, pharmazeutische oder Pflanzenschutzformulierung, enthaltend Alkoxylat-Gemischen gemäß einem der Ansprüche 1 bis 4.

## Claims

1. An alkoxylate mixture comprising alkoxylate of the formula (I)
C₅H₁₁CH(C₃H₇)CH₂O(A)ₙ(B)ₘH (I)
where
A is ethyleneoxy,
B is C₃₋₁₀-alkyleneoxy or mixtures thereof,
where groups A and B may be present in random distribution, alternately or in the form of two or more blocks in any order,
n is a number from 0 to 30,
m is a number from 0 to 20
n + m is at least 1
where
| | |
|---|---|
| 70 to 99% by weight | of alkoxylates A1 in which C₅H₁₁ has the meaning n-C₅H₁₁, and |
| 1 to 30% by weight | of alkoxylates A2 in which C₅H₁₁ has the meaning C₂H₅CH(CH₃)CH₂ and/or CH₃CH(CH₃)CH₂CH₂, |
are present in the mixture.

2. The alkoxylate mixture according to claim 1, wherein C₃H₇ has the meaning n-C₃H₇.

3. The alkoxylate mixture according to claim 1 or 2, wherein, in the formula (I), B is propyleneoxy, n is a number from 1 to 20 and m is a number from 1 to 8.

4. The alkoxylate mixture according to any of claims 1 to 3, wherein 85 to 96% by weight of alkoxylates A1 and 4 to 15% by weight of alkoxylates A2 are present.

5. A process for the preparation of alkoxylate mixtures according to any of claims 1 to 4 by reacting the alcohol mixture with C₂₋₅-alkylene oxides under alkoxylation conditions, it being possible to carry out the alkoxylation in the presence of a double metal cyanide compound as catalyst.

6. The use of alkoxylate mixtures according to any of claims 1 to 4 as emulsifier, foam regulator and as wetting agent for hard surfaces.

7. The use according to claim 6 in detergents, surfactant formulations for the cleaning of hard surfaces, humectants, cosmetic, pharmaceutical and crop protection formulations, paints, coating compositions, adhesives, leather degreasing compositions, formulations for the textile industry, fiber processing, metalworking, food industry, water treatment, paper industry, fermentation or mineral processing and in emulsion polymerizations.

8. A washing, cleaning, wetting, coating, adhesive, leather degreasing, humectant or textile-treatment composition or cosmetic, pharmaceutical or crop protection formulation comprising alkoxylate mixtures as defined in any of claims 1 to 4.

## Revendications

1. Mélanges d'alcoxylates, contenant des alcoxylates de formule générale (I)
C₅H₁₁CH (C₃H₇) CH₂O (A)ₙ(B)ₘH (I)
avec la signification
A éthylèneoxy
B alkylèneoxy en C₃₋₁₀ ou ses mélanges
les groupes A et B pouvant être répartis statistiquement, en alternance ou sous la forme de deux séquences ou plus dans un ordre quelconque,
n nombre de 0 à 30,
m nombre de 0 à 20
n + m au moins 1,
70 à 99 % en poids d'alcoxylates A1, dans lesquels C₅H₁₁ a la signification n-C₅H₁₁, et
1 à 30 % en poids d'alcoxylates A2, dans lesquels C₅H₁₁ a la signification C₂H₅CH(CH₃)CH₂ et/ou CH₃CH(CH₃)CH₂CH₂,
étant présents dans le mélange.

2. Mélanges d'alcoxylates selon la revendication 1, **caractérisés en ce que** C₃H₇ a la signification n-C₃H₇.

3. Mélanges d'alcoxylates selon la revendication 1 ou 2, **caractérisés en ce que** dans la formule générale (I), B signifie propylèneoxy, n est un nombre de 1 à 20 et m est un nombre de 1 à 8.

4. Mélanges d'alcoxylates selon l'une quelconque des revendications 1 à 3, **caractérisés en ce que** 85 à 96 % en poids d'alcoxylates A1 et 4 à 15 % en poids d'alcoxylates A2 sont présents.

5. Procédé de fabrication de mélanges d'alcoxylates selon l'une quelconque des revendications 1 à 4, par réaction du mélange d'alcools avec des oxydes d'alkylène en C₂₋₅ en conditions d' alcoxylation, l'alcoxylation pouvant avoir lieu en présence d'un composé de cyanure de métal double en tant que catalyseur.

6. Utilisation de mélanges d'alcoxylates selon l'une quelconque des revendications 1 à 4 en tant qu'émulsifiants, régulateurs de mousse et agents mouillants pour surfaces dures.

7. Utilisation selon la revendication 6 dans des agents de nettoyage, des formulations de tensioactifs pour le nettoyage de surfaces dures, des agents humidifiants, des formulations cosmétiques, pharmaceutiques et phytosanitaires, des laques, des agents de revêtement, des adhésifs, des agents dégraissants pour le cuir, des formulations pour l'industrie textile, le traitement des fibres, le traitement des métaux, l'industrie agro-alimentaire, le traitement de l'eau, l'industrie du papier, la fermentation ou le traitement des minéraux et pour les polymérisations en émulsion.

8. Agent de lavage, de nettoyage, mouillant, de revêtement, adhésif, dégraissant pour le cuir, humidifiant ou de traitement textile ou formulation cosmétique, pharmaceutique ou phytosanitaire, contenant des mélanges d'alcoxylates selon l'une quelconque des revendications 1 à 4.
